# EUROPEAN PATENT SPECIFICATION

(11) **EP 1 426 066 B1**
(45) Date of publication and mention of the grant of the patent: **17.11.2010**
(21) Application number: 02742644.4
(22) Date of filing: 04.06.2002
(51) Int. Cl.: A61L 27/50, A61F 2/28

(54) **SCAFFOLD PRODUCT FOR HUMAN BONE TISSUE ENGINEERING, METHODS FOR ITS PREPARATION**
GERÜSTPRODUKT FÜR DIE KONSTRUKTION VON MENSCHLICHEM KNOCHENGEWEBE, VERFAHREN ZU SEINER HERSTELLUNG
PRODUIT D'ECHAFAUDAGE DANS LE DOMAINE DU GENIE TISSULAIRE OSSEUX HUMAIN, SES PROCEDES DE PREPARATION

(30) Priority: 05.06.2001 CN 01113076
(43) Date of publication of application: 09.06.2004
(73) Proprietor: Yenssen Biotech Co., Ltd., Jiangyin, Jiangsu 214431 (CN)
(72) Inventor: CHOU, Laisheng, Brookline, MA 02469 (US)
(74) Representative: Meissner, Peter E.
(86) International application number: PCT/CN2002/000389
(87) International publication number: WO 2002/098474

(56) References cited:
- WO-A-01/34060
- WO-A-98/44964
- WO-A-98/52498
- WO-A1-99/07777
- DE-C- 19 610 715
- US-A- 4 103 002
- US-A- 4 192 021
- US-A- 5 017 627
- US-A- 5 552 454
- US-A- 5 977 204
- US-A- 6 051 247

## Description

### FIELD OF THE INVENTION

The present invention relates to scaffolds made of composite materials for human bone tissue engineering, in particular, to scaffolds made of novel medical microparticles composite materials having activity of inducing the regeneration of human bone tissue, methods for its preparation for human bone tissue engineering.

### BACKGROUND OF THE INVENTION

Human bone tissue engineering concerns the process of using absorbable biological materials as scaffolds for inducing the regeneration of autologous bone tissue. The physicochemical properties and the three-dimensional structure of said scaffolds are key factors directly affecting the regeneration of bone tissue. Based on the criterion of molecular compatibility of biological materials, the materials of transplant device for human body or scaffolds for tissue engineering must be safe, and have a bioactivity for inducing the regeneration of relevant human tissues and the restoration of associated physiological functions in cellular and molecular level (Chou, et al, J. Cell Sci., 1995, 108: 1563-1573; Chou, et al, J. Biomed. Mater. Res., 1996, 31:209-217; Chou, et al, J. Biomed. Mater. Res., 1998, 39:437-445).

In the prior art, the combination of materials for scaffolds is mainly selected from natural collagen, calcium phosphate, or organic polymers. Natural collagen has potential disadvantages of higher cost, poorer physical properties, easier to spread diseases and induce hypersensitivity in human body (Pachence and Kohn, Biodegradable polymers for tissue engineering in Principles in Tissue engineering, 1997, p273-293). Calcium phosphate (Kukubo, et al, J. Mater. Science, 1985, 20:2001-2004; Feinberg, et al, Shanghai Journal of Stomatology, 2000, 9:34-38 and 88-93) has the disadvantages of poor retractility, and does show the bioactivity of inducing the regeneration of human bone tissue (Chou, et al, Biomaterials, 1999, 20: 977-985). Organic polymers such as poly(lactic acid) (PLA), poly(glycolic acid) (PGA), or composite of PLA and PGA (PLGA) also have several disadvantages: the acidic degradation products released from the decomposition of said polymer may induce inflammatory reaction and foreign reaction in tissues in the human body, and thus affect the regeneration of bone tissue. Moreover, these polymers have no bioactivity of inducing the regeneration of human bone tissue. (Hubel, Bio/Technology, 1995, 13(6):565-576; Thomson, et al, Polymer scaffolds processing in principles in Tissue Engineering, 1997, p273-293; Cao, et al, Plast Reconstr. Surg. 1997, 100:297-304; Minuth, et al, Cell Tissue Research, 1998, 291(1):1-11; Wong Yulai, et al, Shanghai Journal of Stomatology, June 2000, 9(2):94-96). In the prior art, there are attempts to graft some bioactive proteins, such as cell binding protein or bone inducing protein, on non-active polymer scaffolds (Barrea, et al, Marcromolecules 1995, 28:425-432; Ugo and Reddi, Tissue engineering, morphogenesis, and regeneration of the periodontal tissue by bone morphogenetic proteins, 1997). But these methods can hardly be clinically carried out because of the higher cost, the instability and nonuniformity of the grafted proteins, and the difficulties to sterilize the scaffolds. U.S. Patent 5977204 discloses scaffolds made of a composite material comprising an organic polymer and a bioglass (bioceramics). Said bioglass was firstly disclosed in U.S. Patent 4103002. The combination of silicon, calcium and phosphors was used therein to improve the biocompatibility between said material and human bone tissue, but not to induce the regeneration of bone tissue. In fact, both of U.S. Patent 5977204 and U.S. Patent 4103002 do not definitely describe the activity of silicon to induce the regeneration of bone tissue, nor mention the synergistic inducing effect of calcium and phosphors. Further, the materials disclosed in both patents comprise sodium. However, sodium has no inducing activity on the regeneration of bone tissue. Hence, according to the principles of molecular compatibility of biomaterials, the scaffolds as claimed in U.S. Patent 5977204 does not possess significant bioactivity of inducing the regeneration of bone tissue. In addition, the process as disclosed in said patent uses organic solvents in the preparation of said composite material scaffolds, which may result in potential cytotoxicity to the human body. U.S. Patent 6051247 discloses a composite material comprising the bioglass of U.S. Patent 4103002 and a polysaccharide (such as dextrans) useful in the repairing of bone defects. But said composite material is merely used to form paste or putty, being unsuitable for preparing scaffolds having fine three-dimensional structure and a certain pressure-tolerance for tissue engineering. Further, the bioglass combination of said composite material is inactive to induce the regeneration of bone tissue. The bioglass used in U.S. Patents 5977204, 4103002 and 6051247 has an average particle size(diameter) of greater than 70 microns. The physical properties of the composite materials are obviously affected by such large particles, and the inorganic elements cannot be uniformly released during the decomposition of the composite materials of scaffolds. U.S. Patents 4192021 and 5017627 disclose a composite material comprising an organic polymer and calcium phosphate, which can be used to prepare scaffolds for repairing bone defects. However, this composite material is inactive to induce the regeneration of bone tissue, and the microporosity and pore diameter as designed for said scaffolds are not suitable for the implantation and regeneration of bone cells. U.S. Patent 5552454 discloses a composite material wherein calcium phosphate is coated on the surface of organic polymer particles. This design neither has inducing effect for regeneration of bone tissue, nor can be used to achieve the fine three-dimensional structure of scaffolds for tissue engineering.

The three-dimensional structure of scaffolds for human bone tissue engineering is important for regeneration of both bone tissue and blood vessels in new bone. In the prior art, U.S. Patents 5977204, 4192021, 5017627 and 5552454 all design scaffolds as a uniform, porous or nonporous form, wherein the pore shape, pore size and pore distribution in a porous scaffolds are even. However, such scaffolds with similar and uniformly distributed pores are not suitable for the regeneration of bone tissue. In examples of the use of such scaffolds as disclosed in the prior art, the diameter of the pores in the scaffolds ranges from 150 to 400 microns. It is not large enough to ensure the human cells to enter the central portion of the scaffolds. So the regeneration of bone tissue merely occurs 2 to 3 mm surrounding the scaffolds. In the other aspect, the relatively larger pore diameter (greater than 400 microns) is not suitable for the regeneration of bone tissue (Cartner and Mhiatt, Textbook of Histology, 1997; Tsuruga et al, J. Biochem., 1997, 121:317-324; Gauthier et al, J Biomed. Mat. Res., 1998, 40:48-56). According to the molecular compatibility of biomaterial, the regeneration of blood vessels in the central portion of scaffolds is key for the growth of new bone in the scaffolds, with blood vessels generally formed only in channels having a diameter of greater than 400 microns. Hence, the scaffolds having uniform pores in the prior art cannot meet the different requirements of bone regeneration and blood vessel regeneration simultaneously, and thus the practical application of such scaffolds for bone tissue engineering is limited.

Therefore, there is a great demand in the prior art for scaffolds useful in human tissue engineering, which is bioactive to induce the proliferation and differentiation of human osteoblasts, promote the formation and calcification of new bone, and restore the relevant physiological functions in a cellular and molecular level.

### OBJECT OF THE INVENTION

The object of the present invention is to provide scaffolds being free of organic solvent and having a three-dimensional structure and an external anatomic structure, which are prepared by a hot-cast method without the use of organic solvent based on the principles in molecular compatibility of biomaterials, using a composite microparticulate material made of a combination of silicon, calcium, and phosphorus micro-particles as the bioactive substance of the scaffolds that could actively induce the proliferation and differentiation of human osteoblasts, and promote the formation and calcification of new bone, in combination with an organic polymer at a certain ratio as the carrier, said composite material is bioactive to induce the regeneration of bone tissue and has the desired physical properties. The resulting scaffolds can be used in human bone tissue engineering safely, economically and effectively to repair the defect of bone tissue caused by tumor, inflammation or wound or for orthopedic operation of human bone.

### SUMMARY OF THE INVENTION

To achieve the above objects, one aspect of the present invention is to provide a composite scaffolds for human bone tissue engineering, comprising an organic polymer as carrier with a three-dimensional structure comprising micropores and connecting channels or micropores, characterized by microparticles containing inorganic elements silicone, calcium and phosphorus as bioactive inducing substance, wherein the diameter of the microparticles is less then or equal to 10 microns.

Another aspect of the present invention is to provide a use of inorganic elements silicone, calcium and phosphorus microparticles for the preparation of scaffolds for human bone tissue engineering used in the regenerative repair of bone defect and in the orthopedic operation.

### BRIEF DESCRIPTION OF THE DRAWINGS

- Fig.1: Effects of silicon, calcium, and phosphorus microparticles on the proliferation of osteoblasts, the bioactivity of alkaline phosphatase, the synthesis and secretion of osteocalcin, and the bone calcification in normal human bodies.
- Fig.2: Comparison of performances of the scaffolds made of a composite material comprising silicon, calcium, and phosphorus microparticles and organic polymer (PLGA), and the scaffolds made of single PLGA material, in the induction of proliferation of osteoblasts and the bioactivity of alkaline phosphatase in normal human body.
- Fig.3: Spherical scaffolds made of a composite material comprising silicon, calcium, phosphorus nanoparticles and organic polymer (PLGA).
- Fig.4: SEM photos of micropores of the scaffolds made of a composite material comprising silicon, calcium, phosphorus nanoparticles and organic polymer (PLGA).
- Fig.5: Photos of cylindrical scaffolds having a three-dimensional structure consisting of micropores and connecting channels, which is made of a composite material comprising silicon, calcium, phosphorus nanoparticles and organic polymer (PLGA) by the hot-cast method.
- Fig.6: The animal model for rebuilding the condylar process of human temporomandibular joint by tissue engineering.

### DETAIL DESCRIPTION OF THE INVENTION

The present invention is based on the long-term and intensive study of the present inventor, seeking for microparticulate elements useful as the chemical components for preparing scaffolds for human bone tissue engineering, said microparticulate element is bioactive to induce the regeneration of human bone tissue, auto-degradable and can neutralize the acidic or alkaline substances around the scaffolds in vivo. The present inventors initiatively developed scaffolds for human bone tissue engineering, wherein silicon microparticles are used as the main active ingredient to induce the proliferation and differentiation of human osteoblasts, bone formation and calcification; calcium microparticles are used as an active ingredient to synergistically induce the proliferation and differentiation of osteoblasts; and calcium and phosphorus microparticles are used as active ingredients to synergistically induce the calcification of regenerated bone. These combinations of elements have non-bio-toxicity, are active to induce the regeneration of bone tissue, and can be degraded in vivo and be substituted with new bone. So the use of protein products in tissue engineering is avoided, the production cost is decreased, and the safety and effectiveness of the scaffolds in clinical practices are increased. The bioactive combination of scaffolds of the present invention differs from the bioglass of scaffolds as disclosed in U.S. Patent 5977204 in that the bioactive combination of the present invention comprises only silicon, or comprises silicon as the main component, and a certain proportion of calcium and/or phosphorus, but is free of sodium. In addition, the diameters of the particles of the elements in the present invention are different from that of the prior art. Hence, the present invention relates to the selection of novel chemical substances and combination proportions thereof for regeneration of bone tissue. The present invention uses an organic polymer, such as PLA, PGA or PLGA, as the carrier for said silicon, calcium, phosphorus microparticles, which connects said silicon, calcium and phosphorus microparticles for molding, and provides pressure-resistance for said scaffolds. The silicon, calcium, and phosphorus microparticles in the composite material is used as bioactive components of said scaffolds, and thus said scaffolds serves as a reservoir for said bioactive components. Said bioactive components are released from the scaffolds slowly, continually and uniformly when said organic polymer is degraded in vivo, to induce the formation of bone and to neutralize the acidic decomposition products of said organic polymer, providing an environment suitable for the regeneration of bone tissue. Therefore, the present invention addresses the problems underlying in the prior art that the scaffolds for bone tissue engineering is deficient in biological induction and cannot be used to repair large-volume defects of bone.

In the present invention, all inorganic elements are microparticles, which are different from U.S. Patent 5977204 which relates to elements in the form of particles having a diameter of larger than 50 microns. Unless otherwise stated, the "microparticles" of the present invention is defined as particles having a diameter equal to or less than 10 microns, preferably as nanoparticles having a diameter less than 1000 nm, more preferably less than 100 nm, most preferably between 5 and 80 nm. In the scope of the present invention, the silicon, calcium and phosphorus microparticles having a diameter of larger than 100 nm or smaller than 10 microns can also be used to achieve the purpose of the present invention, as they also have the biological inducing effect.The only differences lie in that such microparticles have a weaker inducing effect because they decompose and diffuse more slowly. The diameter of microparticles as used in the present invention is obviously smaller than those used in the material of scaffolds in the prior art for bone tissue engineering. Further, the diameter of microparticles used in the present invention favours the uniform distribution of chemical elements in the scaffolds, uniform release of said chemical elements from the scaffolds, and can improve the pressure-resistance of the scaffolds.

In the present invention, unless otherwise stated, the "bioactive inducing substance" is defined as a substance that can actively stimulate the normal cell to proliferate and differentiate specifically so as to achieve a specific physiological function. The silicon, calcium and phosphorus elements of the present invention are bioactive inducing substances that can actively induce the normal human osteoblasts to proliferate and stimulate series of specific physiological functions (such as the bioactivity of alkaline phosphatase, the synthesis and secretion of osteocalcin, and calcification of bone) of osteoblasts. All the inorganic element combinations in the scaffolds in the prior art have no biological inducing activity similar to that possessed by the combination of the present invention.

In the present invention, unless otherwise stated, the "scaffolds for human tissue engineering" are defined as scaffolds that have specific three-dimensional structures and shapes consistent with the anatomical morphology of the defect region in the human bone, which is made of a bio-material that is both safe and bioactive, and can be absorbed in vivo within a certain period. When such scaffolds are implanted in vivo, they provide a favorable environmental condition for osteoblasts to proliferate and differentiate, and promote the gradual formation of new bone in the scaffolds, while the frame material of said scaffolds is gradually absorbed and finally disappears in vivo, and the position of said scaffolds is replaced with the new bone. All scaffolds in the prior art are lack of a specific three-dimensional structure similar to that of the scaffolds of the present invention.

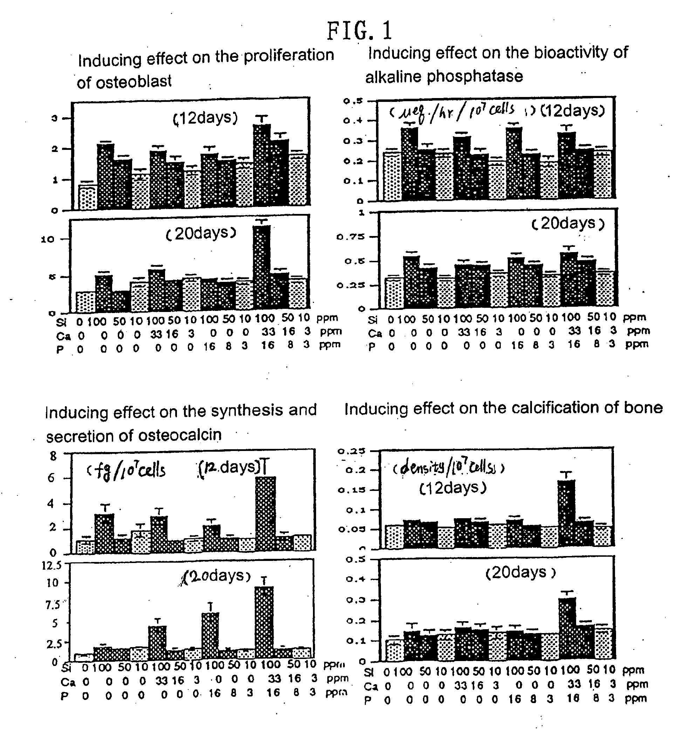

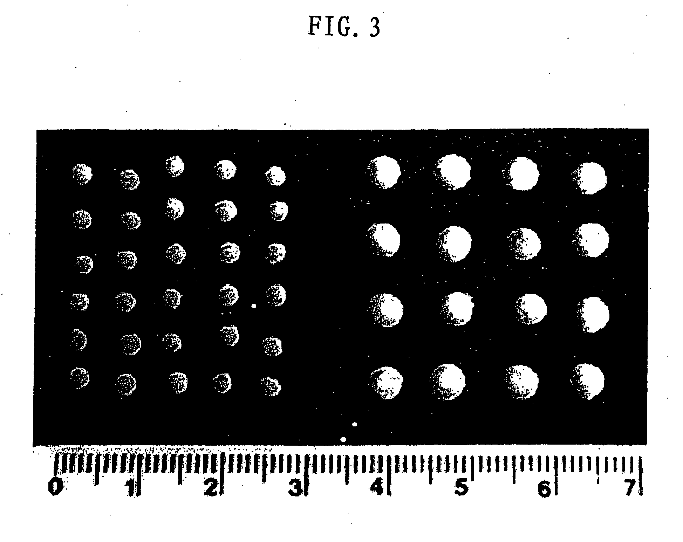

The inventor firstly proves and uses inorganic element "silicon" as the main active substance in scaffolds, which has bio-actively inducing effect in human bone tissue engineering. The experimental data of normal human osteoblasts as shown in Fig.1 (see below) prove that the silicon ions added into the cell culture media have significant inducing and promoting effects(2-4 folds) on the key biological indexes in the formation of new bone, such as the proliferation of osteoblasts, the bioactivity of alkaline phosphatase, the synthesis and secretion of osteocalcin, and the calcification of bone, etc. The data on animal models as shown in Fig.3 (see below) further prove that, after the inorganic element silicon particles are implanted in vivo, inorganic silicon particles diffuse into the surrounding soft tissues, and induce the increase of the concentration of sulfur ion that marks the early stage (two weeks) of the formation of new bone, and also induce the increase of the concentrations of calcium and phosphorus ions that mark the late stage (8 weeks) of the formation of mature and compact bone. Based on these evidences, the present invention firstly achieves a breakthrough, i.e., the specific biological inducing effect of the inorganic element silicon is affirmed, and the element silicon can be used in the scaffolds for bone tissue engineering. In addition, the data of Fig.1 show that the concentration of silicon is directly proportional to the biological inducing effect thereof, and the maximum inducing effect of silicon appears at the saturated concentration of silicon(100 ppm).

In addition, the data of experiments as shown in Fig.1 prove that the combination of the inorganic element silicon and inorganic elements calcium and phosphorus obviously has synergistic effect to promote the proliferation of normal human osteoblasts, the synthesis and secretion of osteocalcin, and the calcification of bone. Hence, the present invention uses inorganic elements calcium and phosphorus as synergistic substances to assist the bioactivity of silicon ions.

In the present invention, unless otherwise stated, all element combinations use silicon ion as the only or main biological inducing substance, and calcium and/or phosphorus as synergistically active substances, so as to actively and effectively induce the formation of new bone tissue. Preferably, the percentages of atomical contents in the "combination of elements used as biological inducing substance" are 60-100% silicon, 0-30% calcium, and 0-20% phosphorus; more preferably, 60-90% silicon, 0-25% calcium, and 0-15% phosphorus; and most preferably, 60-70% silicon, 20-25% calcium, and 10-15% phosphorus.

The silicon/calcium/phosphorus microparticles in said bioactive composite material are in form of a mixture of all sorts of single element microparticles, or are obtained by mixing all sorts of elements and dry grinding by conventional physical or chemical methods. According to Fig.1, the relative amount of the atomical elements in the microparticle mixture or in the microparticles of composit elements is not a vital factor to achieve the purpose of the present invention, because different atomical or weight ratios merely result in different levels of inducing activity. Hence, all combinations with arbitrary atomical or weight ratio of these three elements, wherein silicon is used as main bioactive inducing substance and calcium and phosphorus are used as synergistically bioactive inducing substances, can be used as bioactive substances for the scaffolds of the present invention.

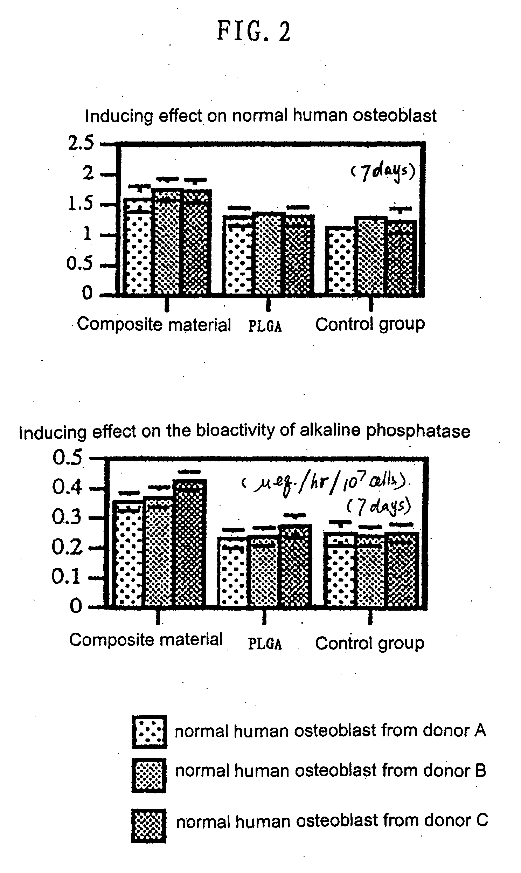

Inorganic elements silicon, calcium and phosphorus are defined as bioactive elements that can induce the proliferation of human bone tissue, the differentiation of osteoblasts, and the calcification of bone. This is also a breakthrough in the biomaterial field. In the prior art, the synthesized or extracted exogenous osteogenin, auxin or connexin and so on are considered as having biological inducing effect, but these biological products have poor safety, inferior bioactive stability, and higher cost, and thus can hardly be used in bioengineering. Besides the above statements, the inducing activity of the inorganic element combinations of the present invention is further proved by the close relationship between the bone regeneration and the distribution of released silicon ion at the interface between the implanted material and the tissue as shown in the animal model of Fig.3, the bone regeneration-inducing effect of the composite material comprising silicon/calcium/phosphorus microparticles and PLGA on the model of human normal osteoblasts as shown in Fig 2, as well as the data on the animal model as shown in Fig.7. For reasons given above, it is firstly proved that inorganic elements silicon, calcium and phosphorus can be used to replace bioactive proteins, and to achieve a significant biological inducing effect. Further, these inorganic elements can be used as bioactive materials in the scaffolds for human bone tissue engineering, so as to obtain a safe and stable scaffolds material that can be prepared easily with lower cost and more safety and stability, and to enhance the practical applicability of the scaffolds.

In the prior art, organic polymers (PLA, PGA and PLGA) are commonly used as single scaffolds material. Yet these organic polymers have no biological inducing activity, and their acidic degradation products in human bodies hinder the regeneration of bone tissue in vivo. In the present invention, the organic polymer is merely used as a carrier for the specific combination of silicon, calcium and phosphorus microparticles. According to the test results on scaffolds having different proportions of carrier, if the content of the inorganic elements combination is greater than 80%, the pressure-resistance of the scaffolds will be relatively weaker, thus a specific steric structure cannot be maintained in animal body, and if the content of the inorganic elements combination is less than 20%, the biological inducing activity will be insufficient to promote the complete formation of new bone within 8 weeks. For making a compromise between the pressure-resistance and the bioactivity of the scaffolds, the present invention defines the volume ratio of silicon/calcium/phosphorus combination to organic polymer in a range from 80:20 to 20:80, preferably from 70:30 to 30:70, according to the biological tests of the examples relating to Fig.2, Fig.4 and Fig.6. Within this range, the solubility of the scaffolds composite can be adjusted. With the increase of the content of silicon/calcium/phosphorus combination, the bioactivity for inducing the regeneration of bone tissue increases. The amounts of these two materials can be adjusted within this range so as to meet the different requirements for the repairation of human bone tissue. The present invention uses a combination of bioactive substances and an organic polymer, forming scaffolds which can serve as the reservoir for such bioactive substances. With the dissolving of the organic polymer (PLA, PGA, PLGA) in vivo (from 1 to 8 weeks), silicon/calcium/phosphorus microparticles are continually and stably released to induce the proliferation and differentiation of osteoblasts, and the formation and calcification of bone during the whole process of bone regeneration. In addition, the released silicon/calcium/phosphorus nanoparticles can neutralize the acidic degradation products of the organic polymer, resulting in an local environment surrounding the scaffolds advantageous for the regeneration of bone tissue.

In the prior art, all calcium phosphates or bioglasses for repair of bone defects are large particles having a diameter greater than 50 microns. If such large particles are used in the composite material, the physical properties of said composite material will be affected. Further, the release of large particles embedded in the organic polymer of the scaffolds is not uniform. Hence, the present invention uses silicon/calcium phosphorus microparticles having a diameter of less than or equal to 10 microns, preferably less than 1000 nm, more preferably less than 100 nm, and most preferably in the range of 5-80 nm, so that said microparticles are embedded evenly in the organic polymer, and are slowly and uniformly released during the degradation of said organic polymer. The microparticles are prepared by mixing microparticles of each of the three elements according to the atomical contents as defined in the present invention.

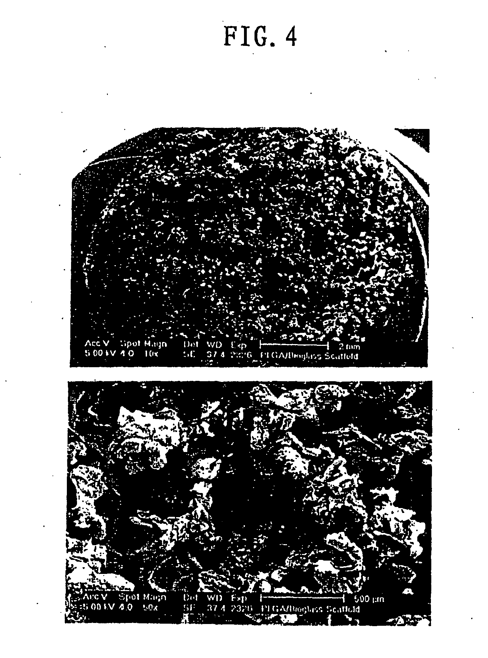

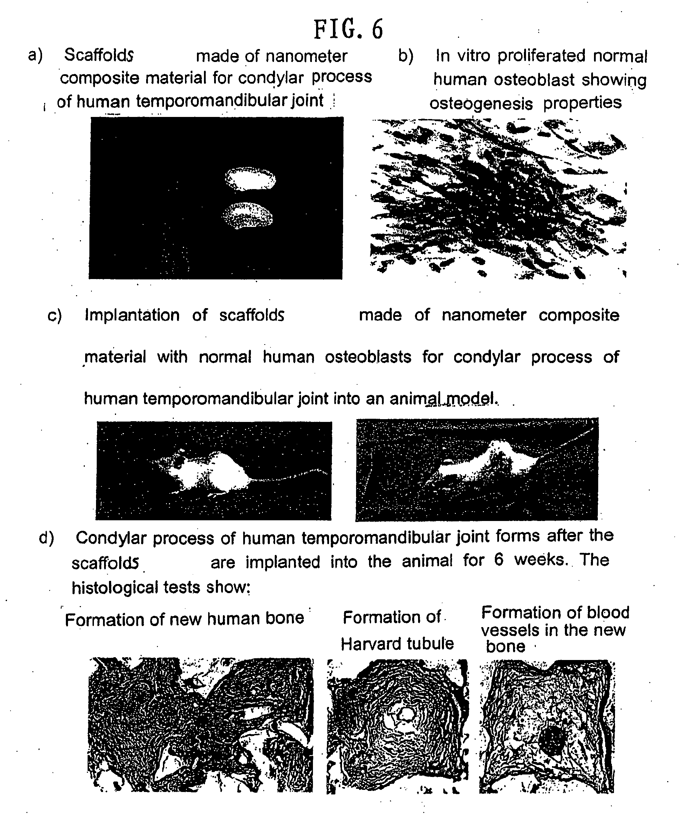

In the prior art, the three-dimensional structure of various scaffolds is microporous with uniform pore diameter and even distribution. The disadvantage of these scaffolds lies in that the relatively smaller micropores (having a diameter less than 300 microns) is adverse to the entry of osteoblasts and blood vessels, and the relatively larger micropores (having a diameter of greater than 400 microns) is adverse to the regeneration of bone tissue. So the practical application of these scaffolds in the bone bioengineering is obviously limited. The present invention uses scaffolds having a three-dimensional structure comprising both micropores and connecting channels as shown in Fig.6 (see below). According to the test results for other diameters, pores with a diameter of less than 100 microns is not suitable for the entry of the cells, and pores with a diameter of greater than 300 microns is not suitable for the formation of new bone. So all scaffolds used in the Examples of preparation and biological tests as shown in Fig.4 to Fig.7 (see below) have micropores with a diameter ranging from 100 to 300 microns. The micropores with a diameter as defined in the present invention is suitable for the proliferation of osteoblasts and regeneration of new bone. The occupancy of micropores of the present invention is from 50% to 90%. For example, the occupancy of micropores of scaffolds used in the Examples as shown in Fig.4, Fig.6 and Fig.7 are 80%, 50% and 50%, respectively. According to the test results for other occupancy of micropores, the physical pressure-resistance of scaffolds with a micropore occupancy of greater than 90% is obviously weaker and insufficient to resist the pressure from surrounding tissues, whereas osteoblasts can hardly enter the scaffolds to form new bone if the occupancy of micropores is less than 50%. According to the test results on different diameters of connecting channels, if the diameter is greater than 500 microns, the pressure-resistance of the scaffolds is obviously weaker, and the neogenesis of large volume-bone tissue is hindered, whereas the entry of cells and the formation of bone tissue are hindered when the diameter is less than 350 microns. Hence, the diameter of the connecting channels of the present invention is in a range from 350 to 500 microns, to ensure the entry of cells into the deep region of the scaffolds and the supply of nutrients and oxygen to new bone through new blood vessels that are grown along said connecting channels into the scaffolds. According to the test results on the intervals between the connecting channels, the pressure-resistance of scaffolds is weaker when the interval is less than 3 mm, while the entry of cells into all micropores of the scaffolds for the formation of new bone is hindered when the interval is greater than 6 mm, thus unsuitable for the formation of new bone. Therefore, the interval between connecting channels of the present invention is preferably in a range from 3 to 6 mm, so as to ensure the uniform entry of cells into all micropores through the connecting channels. The present invention uses combined units with a three-dimensional structure comprising both connecting channels and concentrically arranged micropores, which can be repetitively aggregated (like building blocks) to form various scaffolds of a larger volume for the repairation of large bone defect. This novel three-dimensional structure comprises micropores that are beneficial for the regeneration of bone, and connecting channels that are beneficial for the uniform distribution of cells, the transmission of nutrients for human tissues, and the regeneration of blood vessels in the new bone, and thus can be used to repair a large volume bone defect that cannot be repaired in the prior art. As to a small sized scaffolds having a size less than 5 mm or various bone defects having sclerotic residues of patient, scaffolds having only micropores and various shapes, such as spherical shape, cylindrical shape or quadrate shape as shown in Fig.4 and Fig.6, can be used according to the present invention.

The anatomy shape of the scaffolds of the present invention for human bone tissue engineering can be divided into two groups, namely prefabricated type and tailormade type, depending on the position and size of the bone defect. The prefabricated scaffolds can be in various shapes, such as spherical shape, cylindrical shape, or quadrate shape, etc. When the diameter of the prefabricated scaffolds is less than 5 mm, there will be only micropores in the scaffolds, with no connecting channels. These small sized scaffolds can be of various diameters ranging from 0.5 mm to 5 mm. The prefabricated scaffolds having a size greater than 5 mm are designed as an aggregate of combined units comprising both micropores and connecting channels with different sizes and shapes, so as to fill the space of bone defect to a maxium extent. The prefabricated scaffolds are used to fill bone defect regions with different size and shape and at different locations in human body for the regeneration of bone tissue. The tailormade scaffolds use the human bone scan image as template to design the shape of the scaffolds that fits the anatomical morphology of the human bone, and the scaffolds has a combined structure comprising both micropores and connecting channels, which can be used for repairing large bone defect, for orthopedic operation of human bone, and for treatment of the case without residual bone wall to maintain the shape.

In addition, organic solvents are usually used in the prior art for preparing organic polymer scaffolds for bioengineering. As the organic solvents can hardly be completely removed from the scaffolds, it is harmful to the regeneration of human bone tissue. Unlike the process for preparing the scaffolds for human bone tissue engineering known to the prior art, the process of the present invention uses a conventional hot-cast method to prepare the prefabricated or tailormade scaffolds for bioengineering, avoiding the use of organic solvents. The process of the present invention can avoid the cytotoxicity caused by the residual organic solvent in the scaffolds in the prior art, and can reduce the cost for batch production of the scaffolds.

The clinical use of the scaffolds of the present invention for human bone tissue engineering comprises in-situ implanting cells in vivo, or implanting the in vitro proliferated cells. The in-situ implantation of cells in vivo comprises directly implanting a small sized prefabricated scaffolds into the cavity of human bone defect during surgey, directly using the undifferentiated interstitial cell rich in the blood and tissue exudate entrapped in the cavity of bone defect during the surgey to infiltrate into the space between the pores of the scaffolds, and inducing the regeneration of bone by the material of scaffolds. Thus such a method can be used to repair the defect of bone at unstressed location with residual outer-wall of bone. The implanted prefabricated scaffolds is a combination of scaffolds, with a size of greater than 0.5 mm. For example, the spherical and cylindrical scaffolds as shown in Fig.4 and Fig.6 are used in the aforesaid methods. The implantation of the in vitro proliferated cells is used to repair bone defects at a stressed location or at a location without residual outer-wall of bone. The source of normal human autologous osteoblasts, which is needed in large quantities for repairing a large volume bone defect by implanting the same into scaffolds, is always a severe problem in the medical field. The implantation of in vitro proliferated autologous osteoblasts from normal human as empolyed in the present invention can solve this problem. The present invention uses an autologous superficial skeletal fragment derived from patient as the osteoblast source. A 0.2 cm³ superficial skeletal fragment can proliferate in vitro to produce 6-10 million autologous osteoblast cells having normal osteogenesis activity as shown in Fig.7. Moreover, this leaves no scar, neither functional nor physical influences on the harvesting site. 55 million proliferated osteoblast cells are sufficient to supply the scaffolds for regeneration of a 2 cm³ normal autologous bone. In the clinical practice, the tailormade scaffolds is embedded in a bone defect region after the proliferated osteoblast cells are implanted into said scaffolds in vitro, and the bone is fixed with alloy support splints by normal bone operation. With the regeneration of new bone in the scaffolds, the support force of said splints is gradually reduced, the burden of the new bone is gradually increased, and finally the physiological functions of the regenerated bone are restored (see below, "The animal model for rebuilding the condylar process of human temporomandibular joint by tissue engineering").

As compared to the prior art, the merits of the present invention lie in that: the use of silicon/calcium/phosphorus microparticles having inducing activity on human bone regeneration as bioactive material renders the biological effectiveness of the scaffolds of the present invention significantly superior to those scaffolds known to the prior art without biological inducing activity; the use of combination units comprising both micropores and connecting channels in the scaffolds promotes the uniform distribution of human cells and regeneration of blood vessels in the scaffolds, solving the problem that the regenerated bone is only limited in local region surrounding the scaffolds in the prior art. Moreover, the reparation and regeneration of large human bone defect that cannot be achieved in the prior art now can be achieved in the present invention by repetitively aggregating the combination units of scaffolds with three dimensionally matched structure to form a sufficient volume.

The present invention is further illustrated with the following non-limiting Examples in combination with the Figures.

### Example 1. Silicon, calcium, phosphorus microparticles biologically induce the proliferation of osteoblasts, bioactivity of alkaline phosphatase, synthesis and secretion of osteocalcin, and bone calcification in normal human body significantly

The human osteoblast cells used in the test are obtained from healthy donors aged from 20 to 25 years old. Each of the groups of cells is obtained from 0.2 cm³ superficial skeletal fragments of one donor. There are totally 5 groups of cells used in the test. The mean values and standard deviations of the test data for 5 groups are shown in Fig.1. It can be seen that a 0.2 cm³ superficial skeletal fragment of donors can proliferate to produce 6-10 millions of autologous osteoblast cells having osteogenesis activity in laboratory. The cell culture media used in the test are pre-added with the silicon, calcium, phosphorus particles having a diameter of less than 10 microns at specific concentrations or proportions as shown below in the tables of Fig.1, with the saturation concentration of silicon being 100 ppm. During the culturing, the culture medium with specific concentrations of the particles is replaced with fresh media comprising the particles at the same concentrations every 3 days. On the 12^{th} day and the 20^{th} day, the following tests are carried out: 1) the test of the proliferation of osteoblasts: counting the total number of cells that are growing in culture media with different concentrations or proportions of chemical additives by a conventional cell flow counting machine, and calculating the proliferation folds of osteoblasts as shown in Fig.1 on the basis of the number of cells adhered on the culture dish in the first 24^{th} hours, demonstrating that inorganic element silicon has obvious inducing effect on the proliferation of osteoblasts, and the inducing effect is directly proportional to the concentration of silicon. Moreover, the inorganic elements calcium and phosphorus enhance the biological inducing effect of silicon synergistically; 2) determing the bioactivity of alkaline phosphatase. An important features of normal osteoblass is the secretion of alkaline phosphatase with normal function. The cells cultured under the conditions as shown below in the tables of Fig.1 for 12 days and 20 days are tested; the cells are detached by plasmase, disrupted with a conventional ultrasonic generator, and the resultant cell slurry is analyzed with conventional chromatography; then the micro-equivalent number of the substrate that is degraded by the alkaline phosphatase produced by 10 millions of cells per hour is calculated. The results proved that inorganic element silicon can enhance the bioactivity and inducing effects of alkaline phosphatase proportionally to the concentration of silicon; 3) determining the synthesis and secretion of osteocalcin. The synthesis and secretion of osteocalcin is a specific and important index for the activity of normal human osteoblasts. The content of osteocalcin secreted into the culture media is determined by a conventional immunohistochemical method using a monoclonal antibody against human osteocalcin. The results are expressed as femtogram values of osteocalcin secreted by 10 million cells on the 12^{th} day and on the 20^{th} day. The results demonstrated that inorganic element silicon could significantly induce the increase of the secretion of osteocalcin by the normal human osteoblasts. Such inducing effect is in direct proportional to the concentration of silicon. Moreover, inorganic elements calcium and phosphorus functioned synergistically to assist the biological inducing effect of silicon. And 4) test of bone calcification. The deposition of calcium in the interstice of osteoblasts is one of the important indexes during the final stage of new bone formation. The cells of each group were calcium-stained by conventional methods on the 12^{th} day and 20^{th} day, and the staining density was determined by conventional chromatographic instrument. The results proved that the higher concentrations of silicon, calcium and phosphorus functioned significantly and synergistically to induce and increase the calcification of normal human osteoblasts.

### Example 2. The composite material comprising silicon, calcium, phosphorus microparticles and organic polymer (PLGA), is advantageous over the single PLGA material in inducing the proliferation of normal human osteoblasts and the bioactivity of alkaline phosphatase

This biological assay illustrates the inducing effect of one group of nanometer composite materials of the present invention in cell culture in vitro, and makes a comparison to the single organic polymer PLGA and conventional polystyrene cell culture dishes. The atomical contents of inorganic elements in the element combination of the composite material are 67% silicon, 22% calcium, and 11% phosphorus, and the volume ratio of the inorganic element combination to PLGA is 50:50. This composite material and the single organic polymer PLGA are separately processed to form disks with a diameter of 2 cm and a thickness of 1.5 mm by the hot-cast method with a mould at 200°C for 8 hours (see also example 4 for the detailed steps). The obtained disks are separately placed into conventional polystyrene cell culture dishes with a diameter of 2 cm, cells are innoculated on different molded disks or directly on the conventional polystyrene cell culture dishes without a molded disk, and then the effects of different materials on the cultured cells are determined. The three groups of test cells are obtained from three healthy donors. The proliferation of cells and the bioactivity of alkaline phosphatase are determined by the methods as stated in Fig.1 after said cells are cultured for 7 days. The data as shown in Fig.2 are mean values and mean deviations of these three groups of cells. The results prove that the disc made of the composite material of the present invention has a biological inducing effect superior to that of the single PLGA disc and that of the conventional polystyrene cell culture dish.

### Example 3. The diffusion and distribution of silicon ions after silicon nanometer material is implanted into an animal model, and the ion distribution for inducing the regeneration of new bone tissue

Adult white rabbits are used as animal model in the present biological test. A bone cavity with a diameter of 0.5 cm is made at fibula of the animal model by a bradawl, then the silicon/calcium/phosphorus composite material particles (atomical ratio of Si:Ca:P = 67:22:11) having a diameter of 50-80 nm are filled into said cavity, finally the wounded area is sutured. The test animals are fed for 2 or 8 weeks, and then the portion filled with the composite material and surrounding tissues are removed by a second surgery, which are fixed with 10% formaldehyde, embedded with resin, sectioned as 1 mm slices along the longitudinal section, and finally the ion concentration distributions at two sides of the interface between the region filled with the composite material and the surrounding animal tissue are determined by a radiation ion analyzer. The data shown in Table 1 are mean values of 5 groups of animals in atomical percentage.

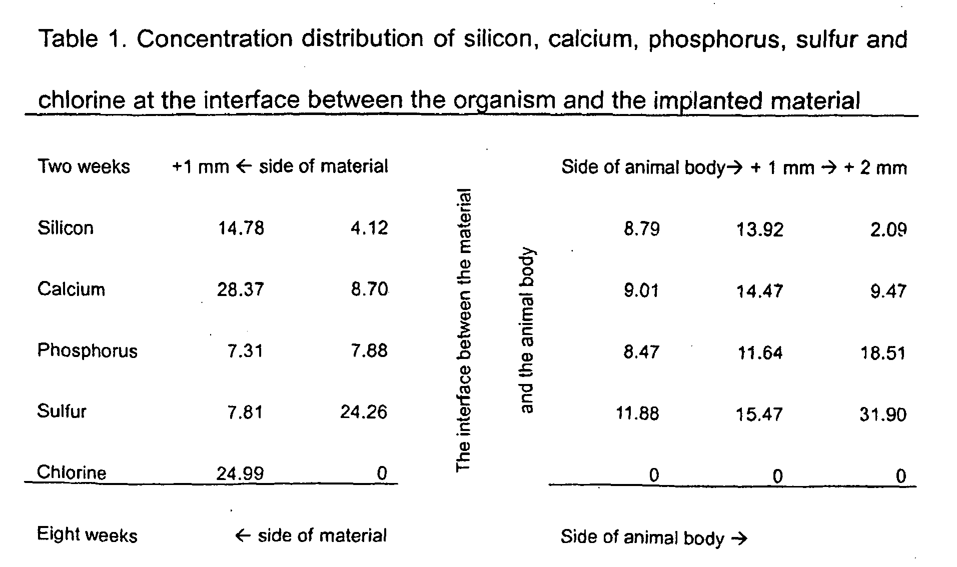

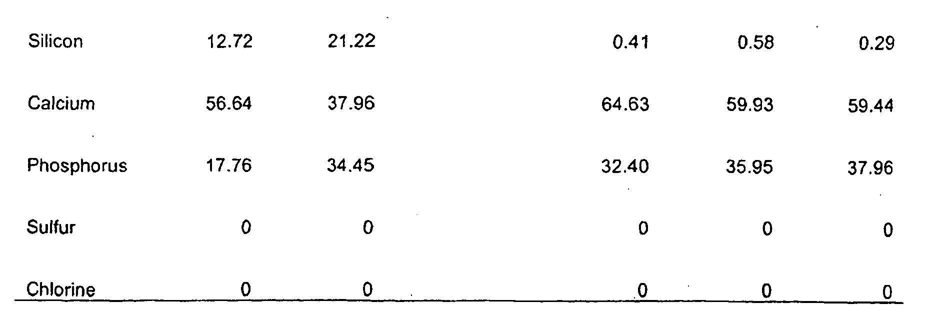

The results show that the silicon ions release from silicon nanoparticles and diffuse into the animal bodies after the composite material is implanted into the animal body for two weeks, resulting in a significant local increase of silicon ion concentration as well as the increase of sulfur ion concentration that indicates the active regeneration of new bone at an early stage. After 8 weeks, the silicon ions disappear in the animal body, and the calcium and phosphorus concentrations that indicate the formation of mature bone increase significantly. This biological test model is also tested histologically, and the results prove that the tissue images at two sides of the interface comply with the dynamic changes of the new bone formation marked by the aforesaid ion distribution changes. The results of this biological test prove that silicon ion has a key bioactive effect on the induction of new bone formation.

### Example 4. Spherical scaffolds made of a composite material comprising silicon, calcium, phosphorus nanoparticles and organic polymer (PLGA) by hot-cast method

This is a preparation example of a spherical scaffolds made of composite material. The starting materials are silica (SiO₂), calcia (CaO), and calcium triphosphate (Ca₅HO₁₃P₃), wherein the atomical contents are 67% silicon, 22% calcium, and 11% phosphorus respectively, and the weight proportions of the starting materials are 40% silica, 6% calcia, and 54% calcium triphosphate correspondingly. The preparation process comprises mixing aforesaid silicon-, calcium- and phosphorus-containing inorganic starting materials according to the said weight proportions, milling by a Retsch track auto-rolling miller for 3 days until the diameter of the microparticles reaches a rang from 5 to 80 nm. The diameter of the microparticles is affirmed by electron scanning microscope. The organic polymer PLGA is milled by a stainless electrical grinding miller, and screened with a fine sieve to obtain PLGA microparticles with a diameter ranging from 25 to 50 microns. The spherical scaffolds as shown in Fig.3 is prepared with the inorganic element combination and PLGA in a ratio of 70:30. A mould is made from polytetrafluoroethylene; then the silicon-, calcium-, phosphorus-containing inorganic starting material microparticles and the organic polymer microparticles are filled into the mould at the aforesaid ratio. After filling, the mould is sintered at 200°C for 8 hours in a ceramic baker, gradually cooled (10°C per minute), and finally demoulded to obtain the spherical scaffolds as shown in Figure 3 (the occupancy of micropores is 80%; and the diameter of the micropores is from 100 to 300 microns).

### Example 5. Images of electron scanning microscope indicate the micropores in the scaffolds made of the composite material comprising silicon, calcium, phosphorus nanoparticles and the organic polymer (PLGA)

The obtained scaffolds made of the composite material comprising silicon, calcium, phosphorus nanoparticles and the organic polymer (PLGA) is cut along its longitudinal section, and its internal micropores are inspected by a conventional electron scanning microscope. The result as shown in Fig.4 proves that the scaffolds prepared by the hot-cast method has a structure with consecutive micropores (the diameter of said micropores ranges from 100 to 300 microns).

### Example 6. Cylindrical scaffolds made of the composite material comprising silicon, calcium, phosphorus nanoparticles and organic polymer (PLGA) by the hot-cast method has a three-dimensional structure comprising both micropores and connecting channels which connect said micropores

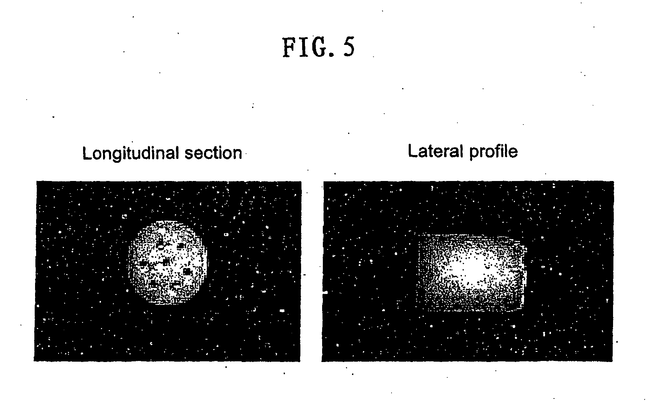

This is an example for the preparation of cylindrical scaffolds made of a composite material. The starting materials are silica (SiO₂), calcia (CaO), and calcium triphosphate (Ca₅HO₁₃P₃), wherein the atomical contents are 67% silicon, 22% calcium, and 11% phosphorus respectively, and the weight proportions of the starting materials are 40% silica, 6% calcia, and 54% calcium triphosphate correspondingly. The preparation process comprises mixing aforesaid silicon-, calcium- and phosphorus-containing inorganic starting materials in said weight proportions, milling by a Retsch track auto-rolling miller for 3 days until the diameter of the microparticles reaches 5 to 80 nm. The diameter of the microparticles is affirmed by electron scanning microscope. The organic polymer PLGA is milled by a stainless electrical grinding miller, and screened by a fine sieve to obtain PLGA microparticles with a diameter ranging from 25 to 50 microns. The prefabricated cylindrical scaffolds as shown in Fig.6 is prepared from the inorganic element combination and PLGA in a ratio of 50:50 as follows. The mould is prepared from polytetrafluoroethylene, and then several stainless steel wires with a diameter of 350-500 microns and an interval of 4 mm are installed in said mould in the designed orientation. The aforesaid starting materials are filled into the mould, sintered at 200°C for 8 hours in a ceramic baker, gradually cooled (10°C per minute), and then demoulded. After removing the cylindrical scaffolds, the stainless steel wires are drawn out, resulting in the cylindrical scaffolds as shown in Figure 5 (the occupancy of micropores is 50%; the diameter of micropores is from 100 to 300 microns; and the diameter of the connecting channels is 500 microns).

### Example 7. The animal model for rebuilding the condylal process of human temporomandibular joint by tissue engineering

As shown in Fig.6a, a polytetrafluoroethylene mould is prepared according to the anatomical shape of the condylar process of human temporomandibular joint, and the tailormade scaffolds is prepared according to the process for preparation of scaffolds shown in Fig.5. As shown in Fig6b, the superficial skeletal fragments are collected from the superficial part of a patient through the following steps: incising the soft tissue at a hidden position of body surface under local anaesthesia, scratching off about 0.2 cm³ of superficial skeletal fragments, and culturing in cell culture media. The incision is sutured. It heals after 3 to 5 days, and there is no effect on the function or shape of the patient. The obtained skeletal fragment is placed in a conventional polystyrene culture dish in a cell culture chamber, and is cultured at 37°C. After 2 weeks, 6 to 10 millions of autologous osteoblasts with normal osteogenesis activity as shown in Fig.6b will be proliferated from the 0.2 cm³ superficial skeletal fragments. Fig.6b indicates the positive results of calcium deposition test by a conventional "Vancusa" method, wherein the brown particles in the accumulation area of pink-stained bone cells are evidences of bone calcification. For reasons given above, these proliferated cells can be used in scaffolds for medical practices. According to the medical practices, 5 millions of cells are sufficient for scaffolds to regenerate and form a 2 cm³ normal autologous bone. The relevant steps comprise detaching the proliferated cells by plasmase from the culture dish, dipping the scaffolds into the cell solution so that the cells enter the portions of scaffolds through the connecting channels and the connected micropores of the scaffolds. The scaffolds with cells therein is implanted into the body of an animal model for test (see Fig.6c) by a conventional surgery. In the clinical practices, the scaffolds implanted into the body is fixed with alloy splints by a conventional bone surgery. With the regeneration of new bone in the scaffolds, the support force of the fixing splints is gradually reduced, the burden of the new bone is gradually increased, and finally the physiological functions of the regenerated bone are restored. As shown in Fig.6d, new bone tissues are formed after the cells and the scaffolds are implanted into the body for 6 weeks. For example, the new bone of this animal model is tested by taking out the implanted scaffolds by surgery, fixing with 10% formaldehyde solution for 24 hours, embedded with paraffin wax, sectioning and staining the tissue by a conventional method. The newly formed normal human bone can be observed under normal optical microscope, and the appearance of Harvard tubule ( 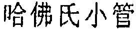 ) proves the formation of high-density bone. In the mean time, newly generated blood vessels are found at the position of the original connecting channels in scaffolds. These histological evidences prove that the neogenesis of the normal bone tissue is satisfactory.

### Reference documents:

1. Barrera DA, Zylstra E, Lansbury PT, Langer R. (1995), Copolymerization and degradation of poly(lactic acid-co-lysine), Macromolecules, 28:425-432.
2. Cao Y, Vacanti JP, Paige KT, Upto J, Vacanti CA. (1997), Transplantation of chondrocytes utilizing a polymer-cell construct to produce tissue-engineered cartilage in the shape of a human ear, Plast. Reconstr. Surg. 100:297-304.
3. Cartner LP, Hiatt JL. (1997), Textbook of Histology, V.B. Saunders Company, Philadephia.
4. Chou L, Firth JD, Uitto V-J, Brunette DM. (1995), Substratum surface topograph alters cell shape and regulates fibronection, mRNA level, mRNA stability, secretion and assembly in human fibroblasts, J. Cell Sci. 108:1563-1573.
5. Chou L, Firth JD, Nathanson D, Uitto V-J, Brunette DM, (1996) Effects of titanium on transcriptional and post-transcriptional regulation of fibronectin in human fibroblasts. J. Biomed. Mater. Res. 31:209-217.
6. Chou L, Firth JD, Uitto V-J, Brunette DM. (1998a), Effects of titanium substratum and grooved surface topography on metalloproteinase-2 expression in human fibroblasts. J. Biomed. Mater. Res. 39:437-445.
7. Chou L, Marek B, Wagner WR. (1999) Effects of hydroxyapatite coating crystallinity on biosolubility, cell attachment efficiency, and proliferation in vitro. Biomaterials, 20:977-985.
8. Feinberg SE, Holloster SJ, Halloran JW, Chu TMG, Krebsbach PH. (2000) A tissue engineering approach to site-specific reconstruction of skeletal structures of the maxillofacial regions. Shanghai Journal of Stomatology. 9:34-38; 88-93.
9. Gauthier AJ, Ducheyne P. Bettiger D. (1999) Effect of surface reaction stage on fibronectin-mediated adhesion of osteoblast-like cells to bioactive glass. J. Biomed. Mat. Res. 40:48-56.
10. Hubbell JA. (1995) Biomaterials in tissue engineering. Bio/Technology. 13(6):565-576.
11. KuKubo T. ITO S, Shigematsu M, Sakka S, Yamamuro T. (1985) Mechanical properties of a new type of apatite-containing glass ceramics for prosthetic application. J. Mater. Science, 20:2001-2004.
12. Minuth WW, Sittinger M, Kloth S. (1998) Tissue engineering: generation of differentiated artificial tissues for biomedical applications. Cell Tissue Research. 291(1):1-11.
13.Pachence JM, Kohn J. (1997) Biodegradable polymers for tissue engineering, in Principles in Tissue Engineering. By Lanz RP, Langer R, Chick WL. Academic Press, London. P273-293.
14.Thomson RC, Yaszemski MJ, Mikos AG. (1997) Polymer scaffolds processing. In Principles in Tissue Engineering. By Lanze RP, Langer R, Chick WL. Academic Press, London. P263-272.
15. Tsuruga E, Hiroko T, Hideaki I, Yuichi W, Yoshinori K. (1997) Pore size of porous hydroxyapatite as the cell-substratum controls BMP-induced osteogenesis. J. Biochem. 121:317-324.
16.Ugo R, Reddi AH. (1997) Tissue engineering, morphogenesis, and regeneration of the periodontal tissue by bone morphogenetic proteins. 8:154-163.
17. Wong Yulai, Cao Yilin, Vacanti, (2000) Experimental studies on condyle of human temporomandibular joint by tissue engineering, Shanghai Journal of Stomatology. Vol.9(2):94-96.
18. U.S. Patent 4103002, July 1978
19. U.S. Patent 4192021, March 1980
20. U.S. Patent 5017627, May 1991
21. U.S. Patent 5552454, September 1996
22. U.S. Patent 5977204, November 1999
23. U.S. Patent 6051247, April 2000

## Claims

1. Scaffolds for human bone tissue engineering with a three-dimensional structure comprising micropores and connecting channels or micropores comprising an organic polymer as carrier **characterized by** microparticles containing inorganic elements silicon, calcium and phosphorus as bioactive inducing substance, wherein the diameter of the microparticles is less then or equal to 10 microns.

2. Scaffolds for human bone tissue engineering according to claim 1, wherein the microparticles are a mixture of silicon microparticles, calcium microparticles and phosphorus microparticles, or are microparticles of a mixture of silicon, calcium and phosphorus elements.

3. Scaffolds for human bone tissue engineering according to claim 1, wherein the diameter of the microparticles is less than 1000 nm

4. Scaffolds for human bone tissue engineering according to claim 3, wherein the diameter of the microparticles, is less than 100 nm.

5. Scaffolds for human bone tissue engineering according to claim 4, wherein the diameter of the microparticles is from 5 to 80 nm.

6. Scaffolds for human bone tissue engineering according to any one of claims 1-5, wherein the atomical contents of the inorganic elements in the microparticles are 60-100 % silicon, less than 30% calcium and less than 20 % phosphorus.

7. Scaffolds far human bone tissue engineering according to claim 6, wherein the atomical contents of the inorganic elements in the microparticles are 60-90 % silicon, less than 25 % calcium and less than 15 % phosphorus.

8. Scaffolds for human bone tissue engineering according to claim 7, wherein the atomical contents of the inorganic clements in the microparticles are 60-70 % silicon, 20-25 % calcium and 10-15% phosphorus.

9. Scaffolds for human bone tissue engineering according to claim 1, wherein the organic polymer as carrier is selected from the group consisting of polyactic acid (PLA), polyglycolic acid (PGA) or composite (PLGA) of PLA and PGA.

10. Scaffolds for human bone tissue engineering according to claim 9, wherein the volume ratio of the microparticles as active components to the organic polymer as carrier is from 80% : 20% to 20% : 80%.

11. Scaffolds for human bone tissue engineering according to claim 10, wherein the volume ratio of the microparticles is from 70% : 30% to 30% : 70%.

12. Scaffolds far human bone tissue engineering according to claim 1, wherein the diameter of the micropores is from 100 to 300 microns.

13. Scaffolds for human bone tissue engineering according to claim 1, wherein the occupancy of the micropores is from 50% to 90%.

14. Scaffolds for human bone tissue engineering according to claim 1, wherein the diameter of the connecting channels is from 350 to 500 microns.

15. Scaffolds for human bone tissue engineering according to claim 1, wherein the intervall between connecting channels is from 3 to 6 mm.

16. Scaffolds for human bone tissue engineering according to claim 1, wherein the connecting channels and the concentric micropores form combined structure units.

17. Scaffolds for human bone tissue engineering according to any one of claims 1-16, wherein the scaffolds are in a prefabricated form or a form tailormade for matching the anatomical morphology.

18. Scaffolds for human bone tissue engineering according to claim 17, wherein the prefabricated form of scaffolds have a shape selected from the group consisting of spherical shape, cylindrical shape and quadrate shape, when the diameter of the prefabricated form of scaffold is less than 5 mm, and the diameter of the micropores varying in a range from 0.5 mm to 5 mm, while the prefabricated form of scaffold having a size greater than 5 mm is an aggregation of combined units comprising both micropores and connecting channels.

19. Scaffolds for human bone tissue engineering according to claim 17, wherein the tailormade scaffold has a shape designed according to the anatomical morphology of the human bone defect as template, and is a large volume scaffolds aggregated with assembling structure units comprising both connecting channels and concentric micropores.

20. Use of inorganic elements silicone, calcium and phosphorus microparticles for the preparation of scaffolds according to claim 1 for human bone tissue engineering used in the regenerative repair of bone defect and in the orthopedic operation.

## Patentansprüche

1. Gerüstsubstanzen für die künstliche Herstellung von menschlichem Knochengewebe (Tissue-Engineering) mit einer dreidimensionalen Struktur aus Mikroporen und Verbindungskanälchen oder -mikroporen, bestehend aus einem organischen Polymer als Trägersubstanz, **gekennzeichnet durch** Mikropartikeln, die als bioaktive Induktionssubstanz die anorganischen Elemente Silizium, Kalzium und Phosphor enthalten, wobei der Durchmesser der Mikropartikeln kleiner oder gleich 10 µm ist.

2. Gerüstsubstanzen für die künstliche Herstellung von menschlichem Knochengewebe nach Anspruch 1, bei denen die Mikropartikeln eine Mischung von Silizium-Mikropartikeln, Kalzium-Mikropartikeln und Phosphor-Mikropartikeln oder Mikropartikeln aus einer Mischung der Elemente Silizium, Kalzium und Phosphor sind.

3. Gerüstsubstanzen für die künstliche Herstellung von menschlichem Knochengewebe nach Anspruch 1, bei denen der Durchmesser der Mikropartikeln kleiner als 1000 nm ist.

4. Gerüstsubstanzen für die künstliche Herstellung von menschlichem Knochengewebe nach Anspruch 3, bei denen der Durchmesser der Mikropartikeln kleiner als 100 nm ist.

5. Gerüstsubstanzen für die künstliche Herstellung von menschlichem Knochengewebe nach Anspruch 4, bei denen der Durchmesser der Mikropartikeln 5 bis 80 nm beträgt.

6. Gerüstsubstanzen für die künstliche Herstellung von menschlichem Knochengewebe nach einem der Ansprüche 1-5, bei denen die Atomgehalte der anorganischen Elemente in den Mikropartikeln 60-100 % Silizium, weniger als 30 % Kalzium und weniger als 20 % Phosphor sind.

7. Gerüstsubstanzen für die künstliche Herstellung von menschlichem Knochengewebe nach Anspruch 6, bei denen die Atomgehalte der anorganischen Elemente in den Mikropartikeln 60-90 % Silizium, weniger als 25 % Kalzium und weniger als 15 % Phosphor sind.

8. Gerüstsubstanzen für die künstliche Herstellung von menschlichem Knochengewebe nach Anspruch 7, bei denen die Atomgehalte der anorganischen Elemente in den Mikropartikeln 60-70 % Silizium, 20-25 % Kalzium und 10-15 % Phosphor sind.

9. Gerüstsubstanzen für die künstliche Herstellung von menschlichem Knochengewebe nach Anspruch 1, bei denen das organische Polymer als Trägersubstanz aus folgender Gruppe gewählt wird: Polymilchsäure (PLA), Polyglykolsäure (PGA) oder Komposit (PLGA) aus PLA und PGA.

10. Gerüstsubstanzen für die künstliche Herstellung von menschlichem Knochengewebe nach Anspruch 9, bei denen das Volumenverhältnis der Mikropartikeln als Aktivkomponenten zum organischen Polymer als Trägersubstanz zwischen 80 % : 20 % und 20 % : 80 % liegt.

11. Gerüstsubstanzen für die künstliche Herstellung von menschlichem Knochengewebe nach Anspruch 10, bei denen das Volumenverhältnis der Mikropartikeln zwischen 70 % : 30 % und 30 % : 70 % liegt.

12. Gerüstsubstanzen für die künstliche Herstellung von menschlichem Knochengewebe nach Anspruch 1, bei denen der Durchmesser der Mikroporen 100 bis 300 µm beträgt.

13. Gerüstsubstanzen für die künstliche Herstellung von menschlichem Knochengewebe nach Anspruch 1, bei denen die Besetzung der Mikroporen zwischen 50 und 90 % liegt.

14. Gerüstsubstanzen für die künstliche Herstellung von menschlichem Knochengewebe nach Anspruch 1, bei denen der Durchmesser der Verbindungskanälchen 350 bis 500 µm beträgt.

15. Gerüstsubstanzen für die künstliche Herstellung von menschlichem Knochengewebe nach Anspruch 1, bei denen der Abstand zwischen den Verbindungskanälchen 3 bis 6 mm beträgt.

16. Gerüstsubstanzen für die künstliche Herstellung von menschlichem Knochengewebe nach Anspruch 1, bei denen die Verbindungskanälchen und die konzentrischen Mikroporen kombinierte Struktureinheiten bilden.

17. Gerüstsubstanzen für die künstliche Herstellung von menschlichem Knochengewebe nach einem der Ansprüche 1-16, bei denen die Gerüstsubstanzen in vorgefertigter Form vorliegen oder passend zur anatomischen Morphologie nach Maß angefertigt sind.

18. Gerüstsubstanzen für die künstliche Herstellung von menschlichem Knochengewebe nach Anspruch 17, bei denen die Gerüstsubstanzen in der vorgefertigten Variante, wenn ihr Durchmesser kleiner als 5 mm ist, entweder eine Kugelform oder eine Zylinderform oder eine Quaderform aufweisen können und der Durchmesser der Mikroporen im Bereich von 0,5 mm bis 5 mm variiert, während vorgefertigte Gerüstsubstanzen, die größer als 5 mm sind, aus kombinierten Einheiten bestehen, die sowohl Mikroporen als auch Verbindungskanälchen umfassen.

19. Gerüstsubstanzen für die künstliche Herstellung von menschlichem Knochengewebe nach Anspruch 17, bei denen die Form der nach Maß angefertigten Gerüstsubstanz entsprechend der anatomischen Morphologie des Knochendefekts als Vorlage gestaltet und die Gerüstsubstanz von größerem Volumen ist und aus zusammengefügten Struktureinheiten besteht, die sowohl Verbindungskanälchen als auch konzentrische Mikroporen umfassen.

20. Verwendung von Mikropartikeln der anorganischen Elemente Silizium, Kalzium und Phosphor zur Herstellung von Gerüstsubstanzen nach Anspruch 1 für das Tissue-Engineering von menschlichem Knochengewebe für die Anwendungsbereiche regenerative Reparatur von Knochendefekten und orthopädische Chirurgie.

## Revendications

1. Matrices pour l'ingénierie tissulaire osseuse comprenant un polymère organique comme support, avec une structure tridimensionnelle comprenant des micropores et des canaux ou micropores de circulation, **caractérisées par** des microparticules contenant les éléments anorganiques silicium, calcium et phosphore comme substance bioactive d'induction, et en ce que le diamètre des microparticules est inférieur ou égal à 10 µm.

2. Matrices pour l'ingénierie tissulaire osseuse selon revendication 1, **caractérisées en ce que** les microparticules sont un mélange de microparticules de silicium, de microparticules de calcium et de microparticules de phosphore, ou des microparticules d'un mélange des éléments silicium, calcium et phosphore.

3. Matrices pour l'ingénierie tissulaire osseuse selon revendication 1, **caractérisées en ce que** le diamètre des microparticules est inférieur à 1000 nm.

4. Matrices pour l'ingénierie tissulaire osseuse selon revendication 3, **caractérisées en ce que** le diamètre des microparticules est inférieur à 100 nm.

5. Matrices pour l'ingénierie tissulaire osseuse selon revendication 4, **caractérisées en ce que** le diamètre des microparticules se situe entre 5 et 80 nm.

6. Matrices pour l'ingénierie tissulaire osseuse selon l'une des revendications 1 à 5, **caractérisées en ce que** la teneur en atomes des éléments anorganiques dans les microparticules est de 60 à 100 % de silicium, inférieure à 30 % de calcium et inférieure à 20 % de phosphore.

7. Matrices pour l'ingénierie tissulaire osseuse selon revendication 6, **caractérisées en ce que** la teneur en atomes des éléments anorganiques dans les microparticules est de 60 à 90 % de silicium, inférieure à 25 % de calcium et inférieure à 15 % de phosphore.

8. Matrices pour l'ingénierie tissulaire osseuse selon revendication 7, **caractérisées en ce que** la teneur en atomes des éléments anorganiques dans les microparticules est de 60 à 70 % de silicium, de 20 à 25 % de calcium et de 10 à 15 % de phosphore.

9. Matrices pour l'ingénierie tissulaire osseuse selon revendication 1, **caractérisées en ce que** le polymère organique comme support est choisi dans le groupe suivant : acide polylactique (PLA), acide polyglycolique (PGA) ou polymorphe (PLGA) composé de PLA et de PGA.

10. Matrices pour l'ingénierie tissulaire osseuse selon revendication 9, **caractérisées en ce que** le rapport volumique entre les microparticules comme composants actifs et le polymère organique comme support se situe entre 80 % / 20 % et 20 % / 80 %.

11. Matrices pour l'ingénierie tissulaire osseuse selon revendication 10, **caractérisées en ce que** le rapport volumique des microparticules se situe entre 70 % / 30 % et 30 % / 70 %.

12. Matrices pour l'ingénierie tissulaire osseuse selon revendication 1, **caractérisées en ce que** le diamètre des micropores se situe entre 100 et 300 µm.

13. Matrices pour l'ingénierie tissulaire osseuse selon revendication 1, **caractérisées en ce que** le taux d'occupation des micropores se situe entre 50 et 90 %.

14. Matrices pour l'ingénierie tissulaire osseuse selon revendication 1, **caractérisées en ce que** le diamètre des canaux de circulation se situe entre 350 et 500 µm.

15. Matrices pour l'ingénierie tissulaire osseuse selon revendication 1, **caractérisées en ce que** l'intervalle entre les canaux de circulation se situe entre 3 et 6 mm.

16. Matrices pour l'ingénierie tissulaire osseuse selon revendication 1, **caractérisées en ce que** les canaux de circulation et les micropores concentriques constituent des unités structurelles combinées.

17. Matrices pour l'ingénierie tissulaire osseuse selon l'une des revendications 1 à 16, **caractérisées en ce que** les matrices se présentent sous forme préfabriquée ou sont réalisées sur mesure en fonction de la morphologie anatomique.

18. Matrices pour l'ingénierie tissulaire osseuse selon revendication 17, **caractérisées en ce que** la forme préfabriquée des matrices présente soit un aspect sphérique, soit un aspect cylindrique ou un aspect carré si le diamètre de la forme préfabriquée est inférieur à 5 mm, et que le diamètre des micropores varie entre 0,5 et 5 mm, tandis que la forme préfabriquée des matrices avec une taille supérieure à 5 mm est un assemblage d'unités combinées qui comprennent des micropores et des canaux de circulation.

19. Matrices pour l'ingénierie tissulaire osseuse selon revendication 17, **caractérisées en ce que** la forme de la matrice réalisée sur mesure est adaptée à la morphologie anatomique du défaut osseux comme modèle, et que les matrices de grande taille sont assemblées en unités combinées qui comprennent des micropores et des canaux de circulation.

20. Utilisation de microparticules des éléments anorganiques silicium, calcium et phosphore pour la fabrication de matrices selon revendication 1 pour l'ingénierie tissulaire osseuse utilisée pour la régénération des défauts osseux et la chirurgie orthopédique.
